Europäisches Patentamt

European Patent Office

Office européen des brevets

(11) Publication number: 0 319 250
A1

## EUROPEAN PATENT APPLICATION

(21) Application number: 88311302.9

(22) Date of filing: 29.11.88

(51) Int. Cl.⁴: G01N 33/92

(30) Priority: 03.12.87 GB 8728310

(43) Date of publication of application:
07.06.89 Bulletin 89/23

(84) Designated Contracting States:
ES GR

(71) Applicant: OXFORD VIROLOGY LIMITED
10 Storey's Gate
Westminster London SW1P 3AY(GB)

Applicant: ST. GEORGE'S HOSPITAL MEDICAL
SCHOOL
Cranmer Terrace Tooting
London SW17 ORB(GB)

(72) Inventor: Knox, Peter
Choppings King's Road
Chalfont St. Giles Buckinghamshire(GB)

(74) Representative: Allam, Peter Clerk et al
LLOYD WISE, TREGEAR & CO. Norman
House 105-109 Strand
London WC2R 0AE(GB)

(54) A method of estimation of systemic cholesterol concentrations from capillary blood samples.

(57) The invention relates to a method of estimating plasma cholesterol levels using small samples of blood and in particular to estimating levels of low-density lipoprotein in capillary blood. The method comprises providing a surface to which a sample of blood is applied, the surface carrying, either over a part or over its whole, anionic groups which have an affinity for low-density lipoprotein. The low-density lipoprotein in the applied blood sample binds to the surface and is subsequently measured by methods which detect either the protein component or the lipid component of the bound low-density lipoprotein.

EP 0 319 250 A1

# A METHOD OF ESTIMATION OF SYSTEMIC CHOLESTEROL CONCENTRATIONS FROM CAPILLARY BLOOD SAMPLES

The invention relates to a method of estimating plasma cholesterol levels using small samples of blood.

Coronary heart disease represents the most common cause of premature death in the Western World and is brought about primarily by the deposition of atherosclerotic plaque within the walls of blood vessels. This deposition occurs at a number of anatomical sites throughout the body but is particularly hazardous in the coronary arteries and carotid vessels.

One of the factors known to predispose a person to the deposition of atherosclerotic plaque and hence atheromatous disease is a high level of circulating cholesterol in the blood. Studies have indicated that although blood cholesterol levels can be influenced by diet many individuals have an inherited genetic predisposition to elevated cholesterol levels and in some cases the condition is so severe that they suffer their first myocardial infarction or heart attack in their early teens.

To a certain extent this condition can be controlled in individuals at risk from artheromatous disease by providing medication to reduce the circulating blood cholesterol and by following the correct diet.

However to improve a patient's prognosis it is necessary to detect the condition in early life so that the appropriate precautions can be taken.

Although the term "plasma or blood cholesterol" is frequently used, there is little if any cholesterol in free form in the blood. Rather the cholesterol, which is predominantly present in an esterfied form, and indeed other lipids such as triglycerides and phospholipids, circulate as complexes bound to proteins. These lipoprotein complexes are divided into four major groups on the basis of density, viz high-density lipoprotein or HDL, low-density lipoprotein or LDL, very low density lipoprotein or VLDL and chylomicrons. HDL has the highest protein to lipid ratio while chylomicrons have the lowest and, in fact, are predominantly lipid.

It is well known that it is with lipoproteins of the LDL class that cholesterol is predominantly associated. Indeed one therapeutic approach which has been proposed for reducing blood cholesterol levels (US-A-4,576,927) is to circulate blood, from the patient's body through a material which selectively absorbs LDL and then reintroduce the cholesterol-reduced blood into the patient. Known absorbants are solid but porous materials which have an anionic polymer linked to their surface for which the protein component of LDL has an affinity.

Thus circulating cholesterol levels correlate with circulating levels of LDL and individuals with high blood cholesterol levels commonly have raised concentrations of LDL. The proteins associated with lipoprotein complexes are called apolipoproteins. The protein present in LDL complexes is specific for the class and is referred to as apoprotein B. Thus raised blood levels of apoprotein B are also an indirect indication of raised cholesterol levels.

The most commonly used method for measurement of plasma cholesterol is an enzyme assay in which cholesterol esterase converts cholesterol esters to free cholesterol. The total cholesterol is then treated with another enzyme, cholesterol oxidase, which generates hydrogen peroxide as one of the reaction products. The concentration of $H_2O_2$ generated may be easily determined by a number of well-known colourimetric methods.

Alternatively the total low-density lipoprotein can be estimated by electrophoresis of plasma in agarose gel. The various lipid-containing bands may be visualized and quantified by staining with any lipid soluble dye.

The total LDL may also be separated from other plasma constituents in any analytical ultracentrifuge or the LDL may be precipitated by the addition to plasma of polyanions, such as for example heparin, which has an affinity for and binds apoprotein B. The cholesterol or protein componants may then be measured by standard methods, for example the cholesterol esterase/oxidase method described above.

The disavantage of all the methods currently available to measure plasma cholesterol is that they involve expensive, time-consuming, laboratory-based assays and require quite large volumes of the patient's blood. Thus it is not practical to routinely screen individuals for high blood cholesterol unless artheromatous disease is suspected. Recent genetic analyses using recombinant DNA technology to identify defective genes which give rise to high blood cholesterol levels suggest that 80% of individuals who are at risk from artheromatous disease are not detected because the current testing procedures are simply not available widely enough. There is a need therefore for a cheap and simple method of estimating blood cholesterol with small volumes of blood. If such a method could be provided much larger sections of the population, (particularly children) could be screened for their predisposition to this life-threatening condition

The method of estimating blood cholesterol in accordance with the invention comprises providing a surface carrying, either over a part or over its whole, anionic groups at a high packing density, said anionic groups having an affinity for low-density lipoprotein, exposing a sample of blood to said surface whereby the low-density lipoprotein component binds to said anionic groups and detecting the bound low-density lipoprotein.

The anionic groups are preferably provided by any anion-containing polymer in which the anions are available to make contact with the low-density lipoprotein when the blood sample is exposed to the polymer and in which the anions are packed sufficiently close together, either over a part of or over the whole length of the polymer chain, to bind the low-density lipoprotein thereto. Examples of suitable polymers are those whose anion generating groups are completely ionised at the pH of blood i.e. about pH 7.4, for instance polymers of carboxylic acids such as polyacrylic acid, polymers of the half-esters or half-amides of dicarboxylic acids e.g. succinic acid, polymers of sulphate half-esters or half-amides, polymers containing sulphate acid groups, polymers of the mono-esters of phosphoric acid and some mucopolysaccharides. Polymers which are particularly preferred in some instances are heparin, heparan sulphate and dextran sulphate because of their high affinity for LDL. Other mucopolysaccharide or anionic polymers which have a relatively low binding affinity for LDL, such as keratan sulphate, chondroitin sulphate, dermatan sulphate and hyaluronic acid are less suitable for use with the method of the invention. Whether or not a particular substrate providing anionic groups is suitable for use herein can be readily determined by experiment.

One particularly interesting class of anion-containing polymers are those which do not require a separate supporting substrate, i.e. those which are of sufficiently anionic character to be able to bind the low density lipoprotein and which can be formed into suitable self-supporting shapes. Nitrocellulose is an example of such an anion-containing polymer since it carries negative charges due to oxidation of the primary alcohol groups of the cellulose during nitration. Further nitrocellulose appears to be able to absorb proteins by other mechanisms as well.

Where a separate supporting substrate is required or desired for the anionic group-containing material, a wide variety of different materials can be used to provide surfaces to carry or support the anionic material. Amongst the suitable substrate materials are proteins; carbohydrates (including agarose, nitrocellulose and other cellulose derivatives); lipids; many organic polymers e.g. polyacrylamide, polymethacrylates, polystyrene, nylons, polyesters, polyethylene oxides, polyglycols, polycarbonates and polyurethanes; latex and polymer latices; silica and silicates; glass; ceramics; and metals and metal oxides. Thus, the supporting substrate may be either porous eg cellulose derivatives, or non-porous.

The anion carrying surface may be planar, tubular or spherical. Thus, the objects which provide suitable support surfaces include strips, slides, capillary tubes or beads.

In embodiments of the invention where a support surface carries a suitable anionic polymer applied thereto, the polymer may be merely absorbed on the surface or chemically coupled to the surface. Chemical coupling between the anionic polymer and the support surface can be brought about by a number of well-known techniques. For example, materials which have high numbers of hydroxyl groups on their surface, such as sepharose, may be activated for coupling to an anionic polymer also carrying amino groups (for example, heparin) by treatment with cyanogen bromide. Alternatively, amino groups may be attached to the surface of materials such as silica and silicates, glass, ceramic, metals and metal oxides by treatment with aminopropyltriethoxysilane and subsequently activated by reaction with glutaraldehyde for coupling to an anionic polymer also carrying amino groups. Methods suitable for effecting chemically coupling between an anionic polymer and other substrate materials are described in the literature. The resulting activated surface may be coated in such a way as to provide a concentration gradient of anions along the surface for a purpose which will be hereinafter described.

Once the sample blood has been exposed to the surface carrying the anions there are a number of ways in which the resulting bound LDL may be detected and quantified. For example the lipid component of the bound LDL may be detected by applying any suitable lipid staining dye. Alternatively the apoprotein B component of the bound LDL can be detected by an immunological technique such as use of fluorescent chromophore conjugated antibodies, radio-actively labelled antibodies or enzyme-linked antibodies. Another possibility is to use the cholesterol esterase/oxidase assay described previously. In a particularly advantageous embodiment of the invention the surface carrying the multiple anionic groups is in the form of beads. Binding of the LDL causes the beads to aggregate together, the degree of aggregation being a measure of the LDL concentration.

In one preferred embodiment of the invention the surface is provided by a glass slide having heparin coupled to its surface following treatment with aminopropyltriethoxysilane and glutaraldehyde.

Once a sample of the patient's blood has been applied to the slide there are two alternative approaches which are preferably then taken to measure the amount of bound LDL. Firstly, the cholesterol or other lipid components of the LDL can be detected by applying a lipid staining dye, eg Sudan black or Oil red O, after first washing the unbound blood components from the slide with a suitable isotonic washing solution. Secondly the bound apoprotein B can be specifically detected by any one of a number of known immunological methods. For example fluorescent chromophore conjugated antibodies to apoprotein B could be applied directly to the bound LDL on the slide. Alternatively radio-actively labelled or enzyme-linked antibodies specific for apoprotein B could be used. A particularly preferred immunological method is to apply an anti-apoprotein B antiserum to the bound LDL followed by enzyme-linked, fluorescent chromophore conjugated or radio-labelled antibodies to anti-apoprotein B antibodies. As an example the apoprotein B antiserum may be raised in rabbits in which case anti-rabbit IgG antibody-conjugates could be used. Antibody conjugates can be prepared from polyclonal sera or nonoclonal antibodies may be used.

Enzyme-linked substrate assays are particularly preferred in which an enzyme conjugated to an appropriate antibody catalyses a reaction which results in an easily detectable, for example, highly coloured product, when substrate is added. Well known suitable enzymes are horseradish peroxide and alkaline phosphatase.

To detect the bound LDL the anti-apoprotein B antiserum is added to the slide following the addition of the blood sample. Antibodies to apoprotein B will bind to the LDL while the remaining antiserum components may be washed off. The enzyme-linked antibodies to the bound apoprotein B antibodies are then applied followed by addition of the enzyme substrate which will be converted to a coloured product, the intensity of which is an indication of bound LDL and hence cholesterol. If desired, for example to be able to measure very low levels of apolipoprotein B, the sensitivity of the enzyme linked assay may be enhanced by replacing the enzyme linked antibodies by avidin linked antibodies, treating the conjugate with a biotin-avidin-enzyme conjugate and developing the color as above.

The concentration of heparin on the slide may be adjusted such that bound LDL is detected, by any of the aforesaid detection methods, only if the LDL concentration in the blood sample is abnormally high, and bearing in mind that the "normal" range of blood cholesterol levels can vary between different ethnic groups.

Alternatively a more quantitative estimate of the LDL concentration may be made by providing a colour comparitor chart correlated to known cholesterol concentrations.

In a second embodiment of the invention, the inner surface of a capillary tube is coupled to heparin. However in this case the tube is prepared so that there is a concentration gradient of heparin along its length. This is achieved by allowing the reagents used to couple heparin to glass to move by capillary action specific distances along the tube. The process is repeated a number of times and each time the distance the reagents move along the tube is increased. Thus a gradient is built up along the tube. A gradation of concentration along the tube of 0.5 µg/ml to 2.0 µg/ml accomplished in steps of 0.5 µg/ml has proved convenient. The tube is then provided with a bulb so that a small sample of blood may be drawn into the tube and immediately expelled. The bound LDL can then be detected by one of the aforementioned methods by drawing the appropriate reagents into the tube.

With this method, an end-point will be obtained at a particular distance along the tube where the heparin concentration is just sufficient for the bound LDL to be detected. The position of the end-point can then be readily compared with the end-points obtained with a standard sample or, as mentioned in connection with the slide assay, a comparitor chart could be prepared indicating the end-point for known LDL concentrations.

In a third embodiment of the invention, the surface is heparin-coated beads. The heparin is coupled to the surface by first treating the bead surface with aminopropyltriethoxysilane and glutaraldehyde or cyanogen bromide depending on the type of material from which the beads are made. When a sample of blood is added to the beads the binding of the LDL to the heparin causes the beads to aggregrate in clumps. The length of time the blood is in contact with the beads before aggregation occurs needs only be a few seconds.

Since the ability of a blood sample to cause the beads to clump depends on the relative concentration of the LDL and anions, the beads may be prepared having a concentration of heparin and hence anions on their surfaces such that they will only aggregate in the presence of blood containing abnormally high levels of LDL but will not be aggregated by the blood of normal individuals. The bead aggregation test therefore provides an almost instantaneous result.

The bead test may be made more quantitative by preparing groups of beads, each group having a different concentration of heparin on their surface, and distributing the groups into the wells of microtitre trays or other suitable receptacles. If a drop of the patients blood is added to each well, an end-point will be detectable in the well where the beads just begin to aggregate. The degree of aggregation can be determined by eye or measured automatically by a known suitable device. The result may be compared

4

with a standard of normal blood or to a suitably prepared chart so that the concentration of LDL, and hence cholesterol, in the test sample can be quantified.

It has been found with the embodiment of the invention involving beads that aggregation is more clearly detectable if the patient's blood is first diluted in a suitable known volume of a suitable isotonic solution, for example phosphate buffered saline and subsequently treated with a detergent such as saponin to lyse the blood cells. However these steps are not essential to the performance to the assay.

The materials and reagents required to perform the method of the invention in any of its embodiments may be provided in the form of a kit.

A kit for estimating blood cholesterol in accordance with the invention comprises a surface carrying, either over a part or over it whole, anionic groups at a high packing density, said anionic groups having an affinity for low-density lipoprotein.

The kit may be provided with lipid staining dyes, or immunological reagents to detect bound low-density lipoprotein. Such a kit may further include a standard sample containing a known concentration of LDL or a comparitor chart which facilitates determination of LDL or cholesterol concentration in a test sample of blood.

The method of determining plasma cholesterol described herein is sufficiently simple that it need not be performed in the laboratory. Rather, patients can be tested in the doctors surgery, for example, with just a 'finger-prick' sample of blood and the result is available immediately. Further, the invention makes it feasible to test the plasma cholesterol level of large number of individuals, particularly children, and so determine their predisposition to artheromatous disease.

The present invention thus represents a considerable improvement over current methods of measuring blood cholesterol because of the simplicity, speed and low cost.

The invention is illustrated by the Examples which follow.

EXAMPLE 1

COVALENT COUPLING OF HEPARIN TO GLASS

Glass tubing, slides or beads were soaked in chromic acid solution (5grms potassium chromate in 100ml sulphuric acid diluted 1.3with water), washed with water followed by 0.1M sodium hydroxide and then allowed to dry in air at room temperature. The glass surface to be coupled with heparin was treated with aminopropyltriethoxysilane for 5 minutes at room temperature. The whole tube or slide was then immersed in single distilled water and washed extensively with stirring, followed by immersion in phosphate buffered saline at room temperature (pH 7.4) for one hour.

The surface to be coupled was then treated with 0.25% glutaraldelyde in phosphate buffered saline (PBS) (pH 7.4) for one hour at room temperature followed by 1ml ethanolamine for 1 hour, and then washed with PBS to remove all the excess glutaraldehyde. The glass surfaces prepared in this way were then treated with different concentrations of heparin to complete the coupling process.

EXAMPLE 2

CYANOGEN BROMIDE COUPLING OF HEPARIN TO SEPHAROSE

Sepharose 4B was washed well with distilled water. The packed beads (5ml) were suspended in distilled water to give a final volume of 8mls and to this was added, with stirring, 2mls of 0.5 molar sodium carbonate (pH 10.5). Cyanogen bromide (1g) was added gradually with stirring, to the sepharose solution together with sodium hydroxide solution in order to keep the pH between 10.5 and 11. The activated sepharose was exhaustively washed with 0.1m sodium citrate (ph 6.5) while gently rocking the slurry of activated sepharose, a solution of heparin in citrate buffer (100 μg/ml) was added and the resulting slurry rocked overnight at 4°C. Ethanolamine (1ml) was added and the slurry rocked for a further hour. The sepharose was finally washed exhaustively with PBS before use.

EXAMPLE 3

Plasma samples of known cholesterol concentration were prepared by ultracentrifuging a bulk plasma sample at a density of 1.2g/ml, removing lipoproteins at the surface of the tube and then mixing different proportions of the lipoprotein-deficient plasma and lipoproteins to generate a series of plasma samples with increasing cholesterol concentration, in this particular case ranging from 2 mM to 17mM in steps of 3mM. The cholesterol concentration in each sample was then estimated using a known enzyme assay.

The standard samples were applied to glass slides having heparin coupled to their surface and the bound LDL detected by an enzyme-linked substrate assay as described herein in which formation of the product was detected by optical density. The results obtained indicated that the optical density correlates with cholesterol concentration in the samples.

EXAMPLE 4

Plasma samples having known cholesterol concentrations were prepared as described in Example 3. The samples were applied to beads in microtitre wells having heparin coupled to their surface and the degree of bead aggregation was measured semi-quantitatively by eye. The results obtained are indicated in the following table in which it is shown that the degree of bead aggregation increases with the cholesterol concentration:

| Cholesterol concentration (mM) | Bead aggregation |
|---|---|
| 1.5 | - |
| 3.0 | - |
| 4.5 | + |
| 6.0 | + |
| 7.5 | + |
| 9.0 | + + + |
| 10.0 | + + + + + |
| 12.0 | + + + + + |
| 13.5 | + + + + + |

EXAMPLE 5

Plasma samples having known concentrations of cholesterol were prepared as described in Example 3. The following manipulations were carried out at intervals of 30 seconds to 2 minutes:

(i) 2 microlitres of each sample were transferred to a nitrocellulose strips;

(ii) The nitrocellulose strip was transferred to a blocking solution composed of normal animal serum and phosphate buffered saline;

(iii) The nitrocellulose strip was transferred to anti-human apolipoprotein-B-enzyme conjugate dissolved in phosphate buffered saline;

(iv) The nitrocellulose strip was transferred to the colour generating substrate dissolved in phosphate buffered saline.

The intensity of the coloured product was seen to correlate with the concentration of cholesterol.

## Claims

1. A method of estimating blood cholesterol, comprising, providing a surface carrying, either over or part of over its whole, anionic groups at high packing density, said anionic groups having an affinity for low-density lipoprotein, exposing a sample of blood to said surface whereby the low-density lipoprotein component binds to said anionic groups and detecting the bound low density lipoprotein.

2. A method as claimed in Claim 1, wherein the anionic groups are provided by a polymer containing anions at sufficient packing density, over at least part of its chain length, to bind low-density lipoprotein.

3. A method as claimed in Claim 2, wherein said polymer itself forms a self-supporting substrate for said anionic groups.

4. A method as claimed in Claim 3, wherein said polymer is nitrocellulose.

5. A method as claimed in Claim 1 or Claim 2, wherein the anions are provided by a polymer of a carboxylic acid, a polymer of a half-ester or half-amide of a dicarboxylic acid, a polymer containing sulphonic acid groups, a polymer of a mono-ester of phosphoric acid or a mucopolysaccharide.

6. A method as claimed in Claim 5, wherein the mucopolysaccharide is heparin, heparan sulphate or dextran sulphate.

7. A method as claimed in any one of Claim 2, 5 or 6, wherein said polymer is supported on and coupled to a separate substrate.

8. A method as claimed in Claim 7, wherein the substrate is formed from a protein, a carbohydrate, a lipid, an organic polymer, a natural or synthetic latex, silica, a silicate, glass, a ceramic, a metal or a metal oxide.

9. A method as claimed in any preceding claim, wherein the surface is planar, tubular or spherical.

10. A method as claimed in Claim 9, wherein the surface is in the form of a strip, a slide, a capillary tube or beads.

11. A method as claimed in Claim 7, wherein the substrate surface to be coupled with the anionic polymer is activated by treatment with cyanogen bromide.

12. A method as claimed in Claim 7, wherein the substrate surface to be coupled with the anionic polymer is activated by sequential treatment with aminopropyltriethoxysilane and glutaraldehyde.

13. A method as claimed in Claim 7, wherein the substrate surface to be coupled with the anionic polymer so as to provide a concentration gradient of anions along the support surface.

14. A method as claimed in any preceding claim, wherein the bound low-density lipoprotein is detected by a lipid-staining dye.

15. A method as claimed in any one of Claims 1-13, wherein the bound low-density lipoprotein is measured by immunological detection of apoprotein B.

16. A method as claimed in Claim 15, wherein apoprotein B is detected by fluorescent chromophore conjugated antibodies.

17. A method as claimed in Claim 15, wherein apoprotein B is detected by radioactively labelled antibodies.

18. A method as claimed in Claim 15, wherein apoprotein B is detected by enzyme-linked antibodies.

19. A method as claimed in any one of Claims 15-18, wherein the method of detection includes the addition of anti-apoprotein B antiserum.

20. A method as claimed in Claim 10, wherein the binding of the low-density lipoprotein causes aggregation of beads.

21. A method as claimed in Claim 20, wherein the anion concentration on the bead surface is such that the beads only aggregate in the presence of abnormally high levels of low-density lipoprotein in a blood sample.

22. A method as claimed in Claim 20, wherein groups of beads are provided, each group having a different concentration of anions of their surface such that the low-density lipoprotein concentration is indicated at the end-point where the beads just begin to aggregate.

23. A method as claimed in any preceding claim, wherein the concentration of anions on the support surface is such that bound low-density lipoprotein is only detectable if the concentration in any blood sample is abnormally high.

24. A kit for estimating blood cholesterol comprising a surface carrying, either over a part or over its whole, anionic groups at a high packing density, said anionic groups having an affinity for low-density lipoprotein.

25. A kit as claimed in Claim 24, comprising also a lipid staining dye to detect the bound low-density lipoprotein.

26. A kit as claimed in Claim 24, comprising also an immunological reagent to detect bound low-density lipoprotein.

27. A kit as claimed in any one of Claims 24-26, comprising also a standard sample with a known concentration of low-density lipoprotein.

28. A kit as claimed in any one of Claims 24-26, comprising also a comparitor chart which facilitates determination of low-density lipoprotein or cholesterol concentrations.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | FR-A-2 343 251 (DEVELOPMENT FINANCE CORP. OF NEW ZEALAND) * Whole document * | 1-3,5,6 ,9,14, 24,25 | G 01 N 33/92 |
| Y | | 15,16, 18,19, 26 | |
| Y,P | US-A-4 722 893 (Y. SHIGETA et al.) * Abstract; column 1, line 1 - column 2, line 9 * | 15,16, 18,19, 26 | |
| X | WO-A-8 101 199 (SYMPHAR S.A.) * Abstract; page 1, line 1 - page 6, line 4; claims * | 1,2,5- 10,24 | |
| Y | | 14,25 | |
| Y | DE-A-2 435 303 (EMVO DIAGNOSTIKA CO. KG) * Claims * | 14,25 | |
| X | EP-A-0 137 221 (BEHRINGWERKE) * Whole document * | 1-3,5,9 ,10,15, 24,26 | TECHNICAL FIELDS SEARCHED (Int. Cl.4) |
| Y | | 6-8 | G 01 N |
| Y | CHEMICAL ABSTRACTS, vol. 102, no. 4, 28th January 1985, pages 473-474, abstract no. 32104c, Columbus, Ohio, US; S. SIDEMAN et al.: "Tailor-made agarose-based reactive beads for hemoperfusion and plasma perfusion", & APPL. BIOCHEM. BIOTECHNOL. 1984, 10, 167-82 * Whole abstract * | 6-8 | C 12 Q |

-/-

The present search report has been drawn up for all claims

| Place of search | Date of completion of the search | Examiner |
|---|---|---|
| THE HAGUE | 24-02-1989 | HITCHEN C.E. |

EPO FORM 1503 03.82 (P0401)

**European Patent Office**

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| A | EP-A-0 031 955 (MILES LABS. INC.) ----- | | |

TECHNICAL FIELDS
SEARCHED (Int. Cl.4)

The present search report has been drawn up for all claims

| Place of search | Date of completion of the search | Examiner |
|---|---|---|
| THE HAGUE | 24-02-1989 | HITCHEN C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)